# EUROPEAN PATENT SPECIFICATION

(11) **EP 2 691 516 B1**
(45) Date of publication and mention of the grant of the patent: **11.05.2016**
(21) Application number: 12717864.8
(22) Date of filing: 28.03.2012
(51) Int. Cl.: C12P 33/00, C12P 33/02, C12N 9/04

(54) **NEW PROCESS FOR THE SELECTIVE OXIDATION OF BILE ACIDS, THEIR SALTS OR DERIVATIVES**
VERFAHREN ZUR SELEKTIVEN OXIDATION VON GALLENSÄURE, IHREN SALZEN ODER IHREN DERIVATEN
PROCÉDÉ D'OXYDATION SÉLECTIVE D'ACIDES BILIAIRES, DE LEURS SELS OU DE LEURS DERIVÉS

(30) Priority: 31.03.2011 IT MI20110534
(43) Date of publication of application: 05.02.2014
(73) Proprietor: PRODOTTI CHIMICI E ALIMENTARI SPA, 15060 Basaluzzo (Alessandria) (IT)
(72) Inventor: MONTI, Daniela, I-22020 Faloppio (co) (IT); FERRANDI, Erica Elisa, I-20861 Brugherio (mb) (IT); RIVA, Sergio, I-20822 Seveso (mb) (IT); POLENTINI, Fausto, I-15067 Novi Ligure (al) (IT)
(74) Representative: Finetti, Claudia
(86) International application number: PCT/IB2012/051476
(87) International publication number: WO 2012/131591

(56) References cited:
- EP-A1- 0 518 661
- EP-A1- 1 114 869
- EP-A1- 1 829 974
- EP-A1- 2 105 500
- CARREA G ET AL: "PREPARATION OF 12 KETOCHENODEOXYCHOLIC-ACID FROM CHOLIC-ACID USING COIMMOBILIZED 12-ALPHA HYDROXYSTEROID DEHYDROGENASE AND GLUTAMATE DEHYDROGENASE WITH NADP CYCLING AT HIGH EFFICIENCY", ENZYME AND MICROBIAL TECHNOLOGY, vol. 7, no. 12, 1 January 1985 (1985-01-01), pages 597-600, XP002364536, STONEHAM, MA, US ISSN: 0141-0229, DOI: 10.1016/0141-0229(85)90027-4
- CARREA G ET AL: "ENZYMATIC PREPARATION OF 12-KETOCHENODEOXYCHOLIC-ACID WITH NADP REGENERATION", BIOTECHNOLOGY AND BIOENGINEERING, vol. 26, no. 5, 1 January 1984 (1984-01-01), pages 560-563, XP002364534, WILEY & SONS, HOBOKEN, NJ, US ISSN: 0006-3592, DOI: 10.1002/BIT.260260526
- FOSSATI ELENA ET AL: "Exploitation of the alcohol dehydrogenase-acetone NADP-regeneration system for the enzymatic preparative-scale production of 12-ketochenodeoxycholic acid", BIOTECHNOLOGY AND BIOENGINEERING, vol. 93, no. 6, 20 April 2006 (2006-04-20) , pages 1216-1220, XP002482706, WILEY & SONS, HOBOKEN, NJ, US ISSN: 0006-3592, DOI: 10.1002/BIT.20753
- Daniela Monti et al: "One-pot multienzymatic synthesis of 12-ketoursodeoxycholic acid: subtle cofactor specificities rule the reaction equilibria of five biocatalysts working in a row", Advanced Synthesis and Catalysis, vol. 351, no. 9 September 2009 (2009-09), pages 1303-1311, XP002666468, DOI: 10.1002/adsc.200800727 Retrieved from the Internet: URL:http://onlinelibrary.wiley.com/doi/10. 1002/adsc.200800727/full
- Pier Paolo Giovannini et al: "7 alpha- and 12 alpha-Hydroxysteroid dehydrogenases from Acinetobacter calcoaceticus lwoffii: a new integrated chemo-enzymatic route to ursodeoxycholic acid", Steroids, vol. 73, no. 14 22 December 2008 (2008-12-22), pages 1385-1390, XP002666469, DOI: 10.1016/j.steroids.2008.06.013 Retrieved from the Internet: URL:http://dx.doi.org/10.1016/j.steroids.2 008.06.013

## Description

The present invention relates to a new process for the industrial production of ursodeoxycholic acid, starting from different bile acids, exemplified by the general formula reported in Scheme 1, salts or derivatives thereof, readily available on the market, such as for example cholic acid (3a,7a,12a-trihydroxy-5β-cholanoic acid, (I)), cholic acid methyl ester (II) and chenodeoxycholic acid (3α,7α-dihydroxy-5β-cholanoic acid, (III)).

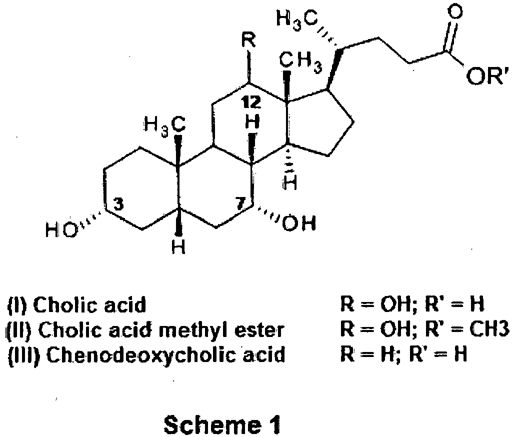

Ursodeoxycholic acid, that is 3α,7β-dihydroxy-5β-cholanoic acid (UDCA), which formula is reported in the following formula (IV) (Scheme 2), 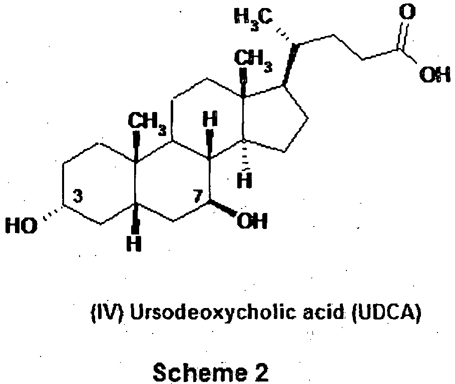 is a compound generally present in small amounts in human bile, where it increases the cholesterol solubilising ability of the bile. UDCA is known for its therapeutic properties: in fact, it is used for the treatment of malfunctions of the liver district, such as dissolution of cholesterol biliary stones, primary biliary cirrhosis and sclerosing cholangitis.

It is therefore of great interest to have at disposal a method for the industrial production of UDCA.

The production of UDCA is currently carried out by chemical synthesis starting from cholic acid extracted from bovine bile, through the formation of the intermediate 12-keto-chenodeoxycholic acid (Hoffmann A.F., Acta Chem. Scand., 1963, 17, 173-186; Sammuelson B., Acta Chem. Scand., 1960, 14, 17-20).

Various synthetic strategies have been suggested, instead of the traditional chemical syntheses of ursodeoxycholic acid, such as for example the one described in Bovara R., Carrea G., Riva S. and Secundo F. Biotechnology letters 1996, 18, 305-308. These strategies use different oxidized derivatives of cholic acid or chenodeoxycholic acid (such as for example 3α,12α-dihydroxy-7-keto-5β-cholanoic acid, 12-keto-chenodeoxycholic acid and 7-keto-lithocholic acid) for the synthesis of UDCA.

Oxidized derivatives of cholic acid and chenodeoxycholic acid can be synthesized both by chemical syntheses, and enzymatic processes.

Chemical processes generally include esterification of the bile acid, selective acetylation of the hydroxyl groups that do not require oxidation, and oxidation of the selected hydroxyl to ketone. However, yields and conversions that can be obtained through synthetic processes are not fully satisfactory from an industrial point of view.

As an alternative to chemical synthesis, the oxidation of cholic acid can be carried out by an enzymatic route using NAD(P)H-dependent hydroxysteroid dehydrogenase enzymatic activities (HSDH) coupled with suitable cofactor regeneration systems, which then can be reacted in catalytic rather than stoichiometric amounts.

HSDH activities show an almost absolute regio- and stereo-selectivity and, therefore, differently from chemical oxidations, the reactions that they catalyze can be conducted without preventive protection of the hydroxyl groups that do not require oxidation.

Since oxidoreduction reactions catalyzed by HSDHs are reversible, the cofactor regeneration system coupled with the oxidation reaction can be exploited not only to reduce the amounts of NAD(P)⁺ to be used, but also to push the reaction equilibrium towards high conversion percentages (Kroutil W., Mang H., Edegger K., Faber K. Adv. Synth. Catal., 2004, 346, 125-142).

Quantitative conversions can be obtained coupling the oxidation reaction with the irreversible reduction reaction of a co-substrate catalyzed by a second dehydrogenase, like in the case of the reductive amination of α-ketoglutaric acid catalyzed by glutamate dehydrogenase in the presence of ammonia. Examples of oxidation reactions of bile acids coupled with glutamate dehydrogenase for the regeneration of the cofactor NAD(P)⁺ are described for example in Riva S., Bovara R., Pasta P., Carrea G. J. Org. Chem., 1986, 51, 2902-2906; Bovara R., Carrea G., Riva S., Secundo F. Biotechnology letters, 1996, 18, 305-308. Such reactions were achieved only with low concentrations of the substrate cholic acid (12.5mM) and cannot be applied on a large scale, due to the high cost of the co-substrate α-ketoglutaric acid and the commercial unavailability of glutamate dehydrogenase for industrial applications.

In the cases where the oxidation reaction is coupled with reversible reduction reactions catalyzed by other dehydrogenases, instead, the use of an excess of co-substrate able to favourably shift the reaction equilibrium towards the desired product is required.

For example, in the oxidation reaction of cholic acid performed with an NADH-dependent 12α-HSDH coupled with the reduction of pyruvic acid to lactic acid catalyzed by an NADH-dependent lactate dehydrogenase (LDH) (Fossati E., Carrea G., Riva S., Polentini F., EP 1 731 618), the co-substrate was used in 100% excess in order to obtain quantitative conversions. Also in this case the high cost of the co-substrate pyruvic acid used for the recycling of the enzyme makes such a process not satisfactory for application on an industrial scale. Further, since NADPH-dependent LDHs are not available, such cofactor regeneration system can be coupled only with oxidations catalyzed by NADH-dependent HSDHs.

Coupling of the oxidation of cholic acid catalyzed by an NADPH-dependent 12α-HSDH with the reduction of acetone through an NADPH-dependent alcohol dehydrogenase (ADH) is described in Fossati E., Polentini F., Carrea G., Riva S., Biotechnol. Bioeng., 2006, 93, 1216-1220. This system is economically advantageous due to the high availability of both coupled enzymes, such as alcohol dehydrogenase, and co-substrate acetone. However, the thermodynamic equilibrium of the coupled process does not allow the quantitative conversion of cholic acid, even in the presence of a large excess of acetone, since both the enzymatic reactions are completely reversible. In optimized conditions, only a 92% conversion of cholic acid in 12-keto-chenodeoxycholic acid was obtained in a reaction mixture containing 25% (volume/volume) acetone.

Recently, the quasi-irreversible biocatalyzed reduction of ketones bearing electron-attractive groups, such as α-halo-ketones or 1,3-diketones, by NAD(P) H-dependent alcohol dehydrogenases has been described (Bisogno F. R., Lavandera I., Kroutil W., Gotor V. J. Org. Chem., 2009, 74, 1730-1732; Lavandera I., Kern A., Resch V., Ferreira-Silva B., Glieder A., Fabian W.M.F., de Wildeman F., Kroutil W., Organic Letters, 2008, 10, 2155-2158). In these works, it is suggested that these reduction reactions can be coupled with the enzymatic resolution of secondary racemic alcohols, such as for example 2-octanol, to have an efficient cofactor regeneration system, used with amounts of co-substrate close to stoichiometric (about 1.5 equivalents).

A new process for the regioselective enzymatic oxidation of bile acids, salts or derivatives thereof, such as cholic acid, chenodeoxycholic acid or cholic acid methyl ester, by NAD(P)⁺-dependent hydroxysteroid dehydrogenases (HSDH) in the presence of an alcohol dehydrogenase (ADH) and methyl acetoacetate as a co-substrate for the regeneration of the cofactor NAD(P)⁺ has now surprisingly been found.

Differently from previously described works where one single ADH reacts both in the oxidation of the substrate of interest, for example 2-octanol, and in the reduction of the sacrificial co-substrate, for example chloroacetone, in this new process, coupling of two different enzymatic activities, HSDH and ADH, was achieved.

This implied the need to identify the optimal reaction conditions for activity and stability of both the enzymes involved in the process, as well as the most suitable conditions to simultaneously ensure solubility and chemical stability of the substrates bile acids and of methyl acetoacetate. In this regard, it is important indeed to underline that bile acids are water soluble in the form of sodium or potassium salts at pHs higher than 7.5-8.0, while methyl acetoacetate is poorly stable at alkaline pHs, particularly at temperatures higher than 25-30°C. Then, the process presented herein was reached by experimentally verifying the influence of different parameters, such as amount of HSDH and ADH enzymes, concentration of the substrates bile acids and of the co-substrate methyl acetoacetate, temperature, and pH, on the degree of conversion of the oxidation reaction.

In particular, NAD(P)⁺-dependent HSDHs used in the process of the present invention preferably have an enzymatic activity ranging from 0.25 to 12 U, where enzymatic activity (U) means the amount of enzyme 7a-HSDH or 12α-HSDH able to transform in one minute one micromole of cholic acid substrate to the corresponding product, oxidized at positions 7 or 12, respectively, in an assay solution containing 12.5mM cholic acid, 0.2mM NAD(P)⁺ and 100mM potassium phosphate buffer, pH 9.0.

The compounds (I), (II) and (III), salts or derivatives thereof are preferably used at a concentration ranging from 0.5% to 4% (weight/volume), that is at a concentration ranging from 12.5mM to 100mM.

The cofactor NAD(P)⁺ + is preferably used in catalytic amounts, at a concentration ranging from 0.2 to 0.8mM.

The co-substrate methyl acetoacetate is used in an amount equal to 2 equivalents referred to each of the compounds (I), (II) or (III). In particular, the co-substrate methyl acetoacetate is used in amounts ranging from 0.3% to 2.4% (volume/volume).

The process of the present invention is preferably carried out at room temperature, that is at about 20°C, and is preferably carried out at pH 8.

Further, while examples from the literature describe reactions exclusively in an aqueous environment, this new process is applicable also to biphasic buffer/organic solvent reaction systems, which can also allow the use of bile acids esters as substrates, such as cholic acid methyl ester.

The cofactor regeneration system used in the process of the present invention is considerably more economic and efficient than previous ones, in that it uses an enzyme and a co-substrate, that is alcohol dehydrogenase and methyl acetoacetate respectively, which are not very expensive and allows the quantitative conversion, i.e. equal to or higher than 99.5%, of bile acids, salts or derivatives thereof such as cholic acid ((I), Scheme 3), cholic acid methyl ester ((II), Scheme 4) and chenodeoxycholic acid ((III), Scheme 5) to the respective selectively oxidized derivatives, depending on the specific HSDH used, that is 7α-HSDH, 12α-HSDH or combinations thereof, on a preparative scale.

In Schemes 3 to 5, the compound shown as (V) is 3α,12α-dihydroxy-7-keto-5β-cholanoic acid, the compound shown as (VI) is 3α,7α-dihydroxy-12-keto-5β-cholanoic acid, the compound shown as (VII) is 3α-hydroxy-7,12-keto-5β-cholanoic acid, the compound shown as (VIII) is 3α,12α-dihydroxy-7-keto-5β-cholanoic acid methyl ester and the compound shown as (IX) is 7-keto-lithocholic acid. Points b) of these schemes describe the reduction reaction of methyl acetoacetate by an NAD(P)H-dependent alcohol dehydrogenase, coupled with the oxidation reactions in the respective points a).

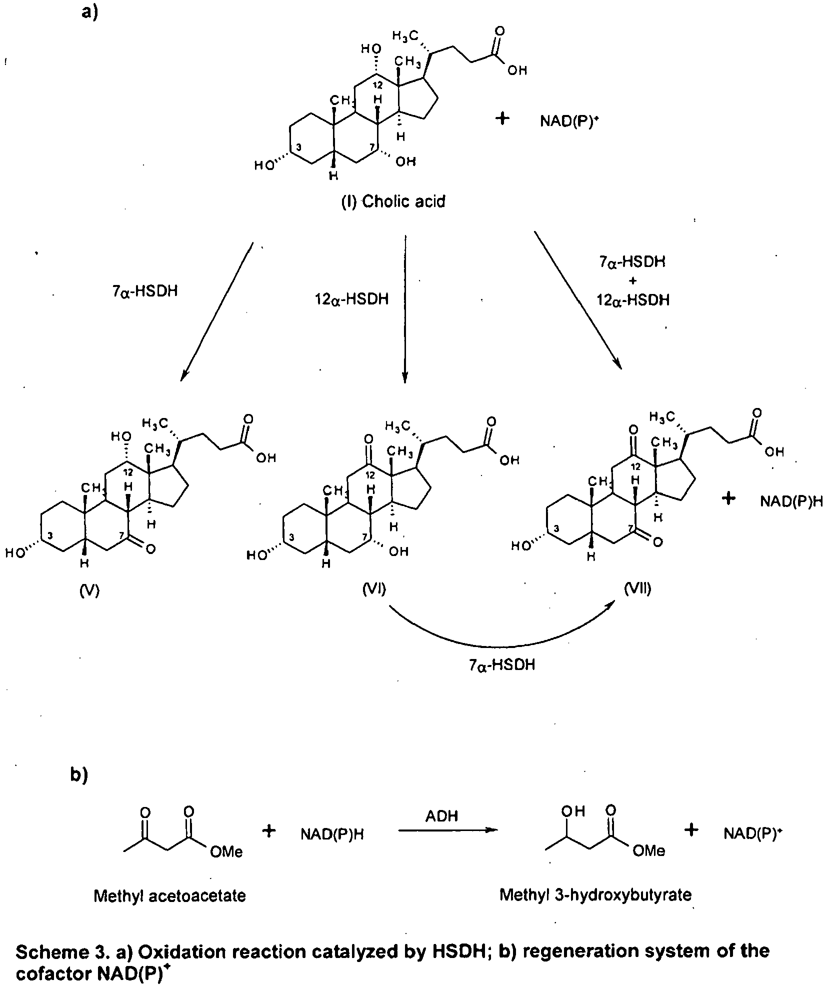

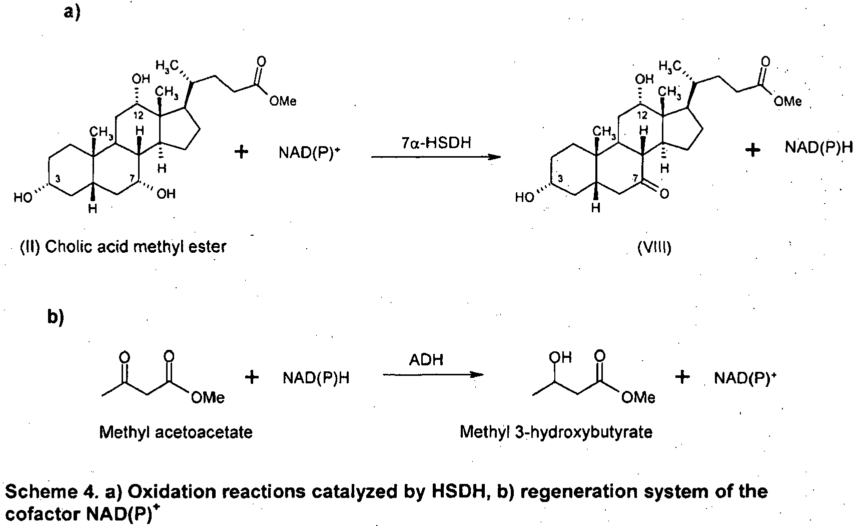

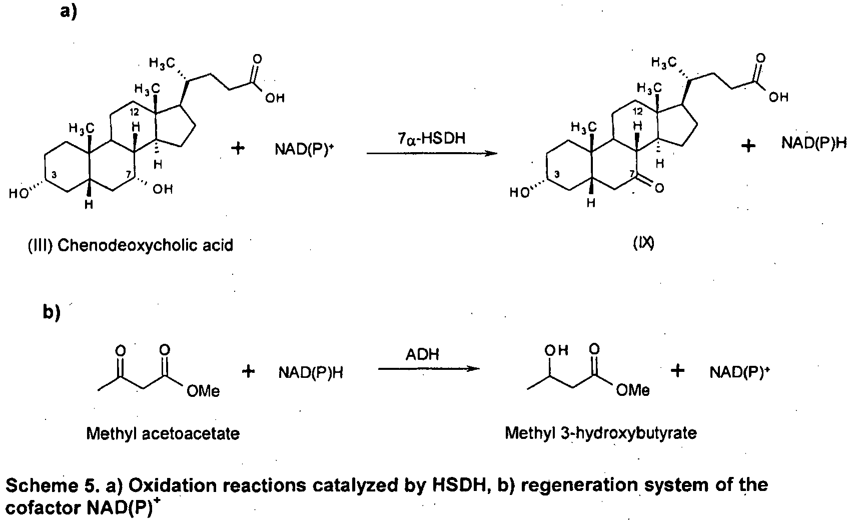

According to an aspect of the process of the invention, the oxidation reaction is conducted in a monophasic system by adding an NAD(P)⁺-dependent hydroxysteroid dehydrogenase (HSDH), such as for example the 12α-HSDH and 7α-HSDH described in Scheme 3 or the 7α-HSDH described in Scheme 5, to an aqueous solution containing cholic acid (I) or chenodeoxycholic acid (III) sodium salts, with catalytic amounts of NAD(P)⁺, which regeneration from NAD(P)H to NAD(P)⁺ is carried out through the conversion of methyl acetoacetate to methyl 3-hydroxybutyrate using an NAD(P)H-dependent alcohol dehydrogenase (ADH).

Depending on the substrate and on the enzymatic activities used, the reactions (oxidations of (I) to the derivatives (V), (VI) and (VII) and of (VI) to the derivative (VII) (Scheme 3), and oxidation of (III) to the derivative (IX) (Scheme 5)) are carried out for a period of time ranging from 4 to 16 h, the substrate bile acid sodium salt is normally used at a concentration ranging from 0.5% to 4% (weight/volume) (that is between 12.5 and 100mM), at pH 8.0 and 20°C, NAD(P)⁺ is used in catalytic amounts, at a concentration ranging from 0.2 to 0.8mM, methyl acetoacetate is usually used in an amount equal to 2 equivalents referred to the bile acid salt, consequently in amounts ranging from 0.3 to 2.4% (volume/volume). The oxidation reaction of the hydroxyl at position 7 of cholic acid methyl ester (II) catalyzed by a 7α-HSDH (Scheme 4) is carried out in a pH 8.0 potassium phosphate buffer/i-propyl acetate biphasic system at 20°C, using the same cofactor regeneration system. The substrate (II) is dissolved at a concentration equal to 4% (weight/volume) (that is 100mM) in i-propyl acetate and added to an equal volume of pH 8.0 potassium phosphate buffer, containing NAD(P)⁺ at a concentration equal to 0.8mM and methyl acetoacetate in an amount equal to 2 equivalents referred to (II), consequently in an amount equal to 2.4% (volume/volume).

The new process described herein is advantageous compared to those previously described for different reasons.

Compared to chemical processes, the new process is simpler and economically advantageous, in that protection of the hydroxyl groups that do not require oxidation is not necessary, thanks to the almost absolute regioselectivity of HSDHs. Further, the process can be carried out in mild reaction conditions (for example at a temperature of 20°C, at atmospheric pressure and at a pH equal to 8.0), which brings both economical and environmental advantages.

Compared to previously described enzymatic processes, it is more economically advantageous due to the use of a reduced excess of co-substrate compared to that usually used with ADHs (for example 2.4% instead of 25% (volume/volume) co-substrate for the oxidation of a 4% (weight/volume) cholic acid solution as described in Fossati E., Polentini F., Carrea G., Riva S., Biotechnol. Bioeng., 2006, 93, 1216-1220). Simultaneously, the new process is more efficient, in that it allows conversions equal to or higher than 99.5%, for thermodynamic reasons not reachable using common ketones, for example acetone, as a co-substrate. Further, the regenerating system of the present invention is more versatile than previous ones, thanks to the availability of both NADH- and NADPH-dependent ADHs developed for applications on an industrial scale, which then can be suitably coupled with HSDH with the same specificity.

The enzymes used in the processes described above can come from a microbial source, such as:
- The NADPH-dependent 7α-HSDH enzyme can come from *Clostridium absonum,* described for example in McDonald I.A., Hutchinson D.M., Forrest T.P., J. Lipid Res., 1981, 22, 458-466, which was cloned and overexpressed in *E. coli* (manuscript in progress).
- The NADH-dependent 7α-HSDH enzyme can be that from *Bacteroides fragilis,* recombinant from *E. coli* Rosetta 2(DE3)pLysS, which is described for example in Zhu D., Stearns J.E., Ramirez M., Hua L., Tetrahedron, 2006, 62, 4535-4539.
- The NADPH-dependent 12α-HSDH enzyme can come from Clostridium sp. marketed by ASA Spezialenzyme GmbH (Germany).
- The NADH-dependent 12α-HSDH enzyme from a microbial source can be that marketed by Genzyme Biochemicals Ltd. (England).
- The NADPH-dependent ADH enzyme can come from *Thermoanaerobacter brockii,* which is described in Peretz M., Bogin O., Tel-Or S., Cohen A., Li G., Chen J. -S., Burstein Y., Anaerobe, 1997, 3, 259-270, or that from a microbial source marketed by SIGMA-ALDRICH.
- The NADH-dependent ADH enzyme can be that from horse liver marketed by SIGMA-ALDRICH.

The oxidized derivatives obtained through the process of the present invention can be used in the synthesis of UDCA ((IV), Scheme 2) by a chemical route, as described in Giordano C., Perdoncin G., Castaldi G., Angew. Chem. Int. Ed., 1985, 24, 499-500; Magni A., Piccolo O., Ascheri A., US 4,834,919; Arosio R., Rossetti V., Beratto S., Talamona A., Crisafulli E., EP 0 424 232 A2; Hattori M., Mikami K., JP 06002184, or alternatively, by an enzymatic route by regio- and stereo-selective reduction of the keto group at 7 catalyzed by a 7β-HSDH, for example as described in Riva S., Bovara R., Pasta P., Carrea G. J. Org. Chem., 1986, 51, 2902-2906; Bovara R., Canzi E., Carrea G., Pilotti A., Riva S., J. Org. Chem., 1993, 58, 499-501; Bovara R., Carrea G., Riva S., Secundo F. Biotechnology letters, 1996, 18, 305-308; Pedrini P., Andreotti E., Guerrini A., Dean M., Fantin G., Giovannini P. P., Steroids, 2006,' 71, 189-198; Monti D., Ferrandi E. E., Zanellato I., Hua L., Polentini F., Carrea G., Riva S., Adv. Synth. Catal., 2009, 351, 1303-1311.

The following examples should be considered as non-limiting illustrations of the invention.

### EXAMPLES

***Example 1 -** Oxidation of the hydroxyl at position* 7 *of cholic acid (I) catalyzed by NADH-dependent* 7α-HSDH *from Bacteroides fragilis in the presence of NADH-dependent ADH from horse liver (SIGMA-ALDRICH).*

The reaction was carried out at 20°C and pH 8.0 in a total volume of 0.5 ml containing: 0.5% (w/v) cholic acid sodium salt at pH 8.0 (12.5mM, obtained diluting a 0.2M cholic acid sodium salt aqueous solution at pH 8.0), 2 equivalents (0.3% (v/v)) of methyl acetoacetate, 12 U of NADH-dependent 7α-HSDH from *Bacteroides fragilis,* recombinant from *E. coli* Rosetta 2(DE3)pLysS, 0.1 U of NADH-dependent ADH from horse liver (SIGMA-ALDRICH), 0.8mM NAD⁺ (40 µl of 10mM NAD⁺ aqueous solution), 50mM potassium phosphate buffer, pH 8.0.

Complete conversion (≥ 99.5%) of cholic acid (I) to 3α,12α-dihydroxy-7-keto-5β-cholanoic acid (V) was obtained in a 16-h period. The degree of conversion was evaluated by TLC analysis using chloroform/methanol/acetic acid (10:1:0.5) as an eluting system.

*Example 2 - Oxidation of the hydroxyl at position 12 of cholic acid (I) catalyzed by NADH-dependent 12*α*-HSDH (Genzyme) in the presence of NADH-dependent ADH from horse liver (SIGMA-ALDRICH).*

The reaction was carried out at 20°C and pH 8.0 in a total volume of 0.5 ml containing: 0.5% (w/v) cholic acid sodium salt at pH 8.0 (12.5mM, obtained diluting a 0.2M cholic acid sodium salt aqueous solution at pH 8.0), 2 equivalents (0.3% (v/v)) of methyl acetoacetate, 4 U of NADH-dependent *12*α*-HSDH* from a microbial source (Genzyme), 0.05 U of NADH-dependent ADH from horse liver (SIGMA-ALDRICH), 0.2mM NAD⁺ (10 µl of a 10mM NAD⁺ aqueous solution), 50mM potassium phosphate buffer, pH 8.0.

Complete conversion (≥ 99.5%) of cholic acid (I) to 3α,7α-dihydroxy-12-keto-5β-cholanoic acid (VI) was obtained in a 16-h period. The degree of conversion was evaluated by TLC analysis using chloroform/methanol/acetic acid (10:1:0.5) as an eluting system.

*Example 3 - Oxidation of the hydroxyl* at *position 12 of cholic acid (I) catalyzed by NADPH-dependent 12*α*-HSDH (ASA) in the presence of NADPH-dependent ADH from T. brockii (SIGMA-ALDRICH).*

The reaction was carried out at 20°C and pH 8.0 in a total volume of 0.5 ml containing: 0.5% (w/v) cholic acid sodium salt at pH 8.0 (12.5mM, obtained diluting a 0.2M cholic acid sodium salt aqueous solution at pH 8.0), 2 equivalents (0.3% (v/v)) of methyl acetoacetate, 4 U of NADPH-dependent *12*α*-HSDH* (ASA), 10 U of NADPH-dependent ADH from *T. brockii* (SIGMA-ALDRICH), 0.2mM NADP⁺ (10 µl of a 10mM NADP⁺ aqueous solution), 50mM potassium phosphate buffer, pH 8.0. Complete conversion (≥ 99.5%) of cholic acid (I) to 3α,7α-dihydroxy-12-keto-5β-cholanoic acid (VI) was obtained in a 16-h period. The degree of conversion was evaluated by TLC analysis using chloroform/methanol/acetic acid (10:1:0.5) as an eluting system.

***Example 4 -** Oxidation of the hydroxyl at position 7 of cholic acid (I) catalyzed by NADPH-dependent 7α-HSDH from C. absonum in the presence of NADPH-dependent ADH, recombinant from E. coli (SIGMA-ALDRICH).*

The reaction was carried out at 20°C and pH 8.0 in a total volume of 0.5 ml containing: 4% (w/v) cholic acid sodium salt at pH 8.0 (100mM, obtained diluting a 0.2M cholic acid sodium salt aqueous solution at pH 8.0), 2 equivalents (2.4% (v/v)) of methyl acetoacetate, 0.25 U of NADPH-dependent 7α-HSDH from *C*. *absonum,* recombinant from *E*. *coli,* 4 U of NADPH-dependent ADH, recombinant from *E. coli* (SIGMA-ALDRICH), 0.8mM NADP⁺ (40 µl of a 10mM NADP⁺ aqueous solution), 50mM potassium phosphate buffer, pH 8.0.

Complete conversion (≥ 99.5%) of cholic acid (I) to 3α,12α-dihydroxy-7-keto-5β-cholanoic acid (V) was obtained in a 16-h period. The degree of conversion was evaluated by TLC analysis using chloroform/methanol/acetic acid (10:1:0.5) as an eluting system.

***Example 5 -** Oxidation of the hydroxyl at position* 7 *of cholic acid (I) catalyzed by NADPH-dependent 7α-HSDH from C. absonum in the presence of NADPH-dependent ADH from T. brockii (SIGMA-ALDRICH).*

The reaction was carried out at 20°C and pH 8.0 in a total volume of 0.5 ml containing: 0.5% (w/v) cholic acid sodium salt at pH 8.0 (12.5mM, obtained diluting a 0.2M cholic acid sodium salt aqueous solution at pH 8.0), 2 equivalents (0.3% (v/v)) of methyl acetoacetate, 4 U of NADPH-dependent 7α-HSDH from *C*. *absonum,* recombinant from *E. coli,* 10 U of NADPH-dependent ADH from *T. brockii* (SIGMA-ALDRICH), 0.2mM NADP⁺ (10 µl of a 10mM NADP⁺ aqueous solution), 50mM potassium phosphate buffer, pH 8.0.

Complete conversion (≥ 99.5%) of cholic acid (I) to 3α,12α-dihydroxy-7-keto-5β-cholanoic acid (V) was obtained in a 4-h period. The degree of conversion was evaluated by TLC analysis using chloroform/methanol/acetic acid (10:1:0.5) as an eluting system.

***Example 6 -** Oxidation of the hydroxyl at position 7 of 12-keto-chenodeoxycholic acid (VI) catalyzed by NADPH-dependent 7α-HSDH from C. absonum in the presence of NADPH-dependent ADH, recombinant from E. coli (SIGMA-ALDRICH).*

The reaction was carried out at 20°C and pH 8.0 in a total volume of 0.5 ml containing: 4% (w/v) 12-keto-chenodeoxycholic acid sodium salt at pH 8.0 (100mM, obtained diluting a 0.2M 12-keto-chenodeoxycholic acid sodium salt aqueous solution at pH 8.0), 2 equivalents (2.4% (v/v)) of methyl acetoacetate, 4 U of NADPH-dependent 7α-HSDH from *C*. *absonum,* recombinant from *E*. *coli,* 4 U of NADPH-dependent ADH, recombinant from *E*. *coli* (SIGMA-ALDRICH), 0.8mM NADP⁺ (40 µl of a 10mM NADP⁺ aqueous solution), 50mM potassium phosphate buffer, pH 8.0.

Complete conversion (≥ 99.5%) of 12-keto-chenodeoxycholic acid (VI) to 3α-hydroxy-7,12-diketo-5β-cholanoic acid (VII) was obtained in a 16-h period. The degree of conversion was evaluated by TLC analysis using chloroform/methanol/acetic acid (10:1:0.5) as an eluting system.

*Example 7* - *Oxidation of the hydroxyls at positions 7 and 12 of cholic acid (I) catalyzed by NADPH-dependent 7α-HSDH from C. absonum and NADPH-dependent 12α-HSDH (ASA) in the presence of NADPH-dependent ADH, recombinant from E. coli (SIGMA-ALDRICH).*

The reaction was carried out at 20°C and pH 8.0 in a total volume of 0.5 ml containing: 0.5% (w/v) cholic acid sodium salt at pH 8.0 (12.5mM, obtained diluting a 0.2M cholic acid sodium salt aqueous solution at pH 8.0), 2 equivalents (0.6% (v/v)) of methyl acetoacetate, 2 U of NADPH-dependent 7α-HSDH from C. *absonum* recombinant from *E*. *coli,* 4 U of NADPH-dependent 12α-HSDH (ASA), 4 U of NADPH-dependent ADH recombinant from *E. coli* (SIGMA-ALDRICH), 0.8mM NADP⁺ (40 µl of a 10mM NADP⁺ aqueous solution), 50mM potassium phosphate buffer, pH 8.0.

Complete conversion (≥ 99.5%) of cholic acid (I) to 3a-hydroxy-7,12-diketo-5β-cholanoic acid (VII) was obtained in a 16-h period. The degree of conversion was evaluated by TLC analysis using chloroform/methanol/acetic acid (10:1:0.5) as an eluting system.

***Example 8 -** Oxidation of the hydroxyl at position 7 of chenodeoxycholic acid (III) catalyzed by NADPH-dependent* 7α-HSDH *from C. absonum in the presence of NADPH-dependent ADH, recombinant from E. coli (SIGMA-ALDRICH).*

The reaction was carried out at 20°C and pH 8.0 in a total volume of 0.5 ml containing: 2% (w/v) chenodeoxycholic acid sodium salt at pH 8.0 (50mM, obtained diluting a 0.2M chenodeoxycholic acid sodium salt aqueous solution at pH 8.0), 2 equivalents (1.2% (v/v)) of methyl acetoacetate, 4 U of NADPH-dependent 7α-HSDH from *C*. *absonum,* recombinant from *E*. *coli,* 3 U of NADPH-dependent ADH, recombinant from *E*. *coli* (SIGMA-ALDRICH), 0. 6mM NADP⁺ (30 µl of a 10mM NADP⁺ aqueous solution), 50mM potassium phosphate buffer, pH 8.0.

Complete conversion (≥ 99.5%) of chenodeoxycholic acid (III) to 7-keto-lithocholic acid (IX) was obtained in a 16-h period. The degree of conversion was evaluated by TLC analysis using chloroform/methanol/acetic acid (10:1:0.5) as an eluting system.

***Example 9 -** Oxidation in a biphasic system of the hydroxyl at position 7 of cholic acid methyl ester (II) catalyzed by NADPH-dependent 7α-HSDH from C. absonum in the presence of NADPH-dependent ADH, recombinant from E. coli (SIGMA-ALDRICH).*

The reaction was carried out at 20°C, pH 8.0 and 1400 rpm in a total volume of 1 ml containing: a) 0.5 ml of a 4% *i-propyl* acetate solution (w/v) of cholic acid methyl ester (100mM, obtained dissolving 20 mg of cholic acid methyl ester in 0.5 ml of *i-propyl acetate*); b) 0.5 ml of 50mM potassium phosphate buffer, pH 8.0, in turn containing 2 equivalents (2.4% (v/v)) of methyl acetoacetate, 5.5 U of NADPH-dependent 7α-HSDH from *C*. *absonum* recombinant from *E. coli,* 4 U of NADPH-dependent ADH recombinant from *E. coli* (SIGMA-ALDRICH), 0.8mM NADP⁺ (40 µl of a 10mM NADP⁺ aqueous solution).

Complete conversion (≥ 99.5%) of cholic acid methyl ester (II) to 3α,12α-dihydroxy-7-keto-5β-cholanoic acid methyl ester (VIII) was obtained in a 16-h period. The degree of conversion was evaluated by TLC analysis using chloroform/methanol/acetic acid (10:1:0.5) as an eluting system.

## Claims

1. A process for the regioselective enzymatic oxidation at positions 7 and/or 12 of compounds of formula 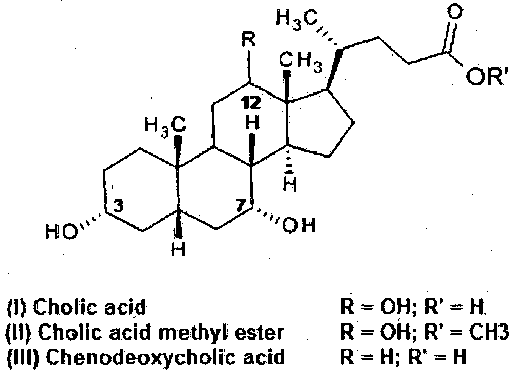 or salts or derivatives thereof, comprising reacting said compounds with NAD(P)⁺-dependent hydroxysteroid dehydrogenase (HSDH), in the presence of a regeneration system of the co-factor NAD(P)⁺ comprising methyl acetoacetate and NAD(P)⁺-dependent alcohol dehydrogenase (ADH).

2. The process according to claim 1, wherein the NAD (P)⁺-dependent HSDH has an enzymatic activity ranging from 0.25 to 12 U.

3. The process according to claim 1, wherein the compounds having formula (I), (II) or (III), salts or derivatives thereof have a concentration ranging from 0.5% to 4% (weight/volume).

4. The process according to claim 1, wherein the compounds having formula (I), (II) or (III), salts or derivatives thereof have a concentration ranging from 12.5mM to 100mM.

5. The process according to any one of preceding claims, wherein the cofactor NAD(P)⁺ is used in catalytic amounts, at a concentration ranging from 0.2mM to 0.8mM.

6. The process according to any one of preceding claims, wherein the co-substrate methyl acetoacetate is used in an amount equal to 2 equivalents referred to each of compounds (I), (II) and (III).

7. The process according to any one of preceding claims, wherein the co-substrate methyl acetoacetate is used in amounts ranging from 0.3% to 2.4% (volume/volume).

8. The process according to any one of preceding claims, wherein the oxidation is quantitative, i.e. higher than or equal to 99.5%.

9. The process according to any one of preceding claims, carried out at room temperature, i.e. at about 20°C.

10. The process according to any one of preceding claims, carried out at a pH equal to 8.

11. The process according to claim 10, further comprising a buffer system, preferably consisting of potassium phosphate buffer.

12. The process according to claim 1, **characterized in that** it is carried out in an aqueous/organic biphasic system, wherein the organic solvent is preferably i-propyl acetate.

13. The process according to claims 11 and 12, wherein the buffer system is potassium phosphate buffer and the organic solvent is i-propyl acetate.

14. The process according to claim 1, wherein the cholic acid or chenodeoxycholic acid salt is sodium salt.

15. A process for the preparation of ursodeoxycholic acid (IV), **characterized in that** it comprises a process for the synthesis of 3α,12α-dihydroxy-7-keto-5β-cholanoic acid (IV) according to any one of claims 1 to 14.

16. A process for the preparation of ursodeoxycholic acid (IV), **characterized in that** it comprises a process for the synthesis of 3α,7α-dihydroxy-12-keto-5β-cholanoic acid (VI) according to any one of claims 1 to 14.

17. A process for the preparation of ursodeoxycholic acid (IV), **characterized in that** it comprises a process for the synthesis of 3α-hydroxy-7,12-keto-5β-cholanoic acid (VII) according to any one of claims 1 to 14.

18. A process for the preparation of ursodeoxycholic acid (IV), **characterized in that** it comprises a process for the synthesis of 7-keto-lithocholic acid (IX) according to any one of claims 1 to 14.

19. A process for the preparation of ursodeoxycholic acid (IV), **characterized in that** it comprises a process for the synthesis of 3α,12α-dihydroxy-7-keto-5β-cholanoic acid methyl ester (VIII) according to any one of claims 1 to 13.

## Patentansprüche

1. Verfahren zur regioselektiven, enzymatischen Oxidation in Position 7 und/oder Position 12 von Verbindungen mit der Formel 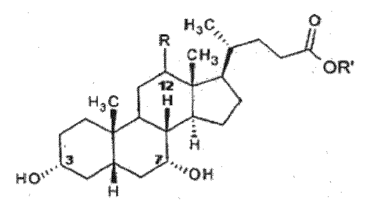
(I) Cholsäure R = OH; R' = H
(II) Cholsäuremethylester R = OH; R' = CH3
(III)Chenodesoxycholsäure R = H; R' = H oder deren Salzen oder Derivaten, umfassend das Umsetzen der Verbindungen mit NAD(P)⁺-abhängiger Hydroxysteroid-Dehydrogenase (HSDH), in Gegenwart eines Regenerationssystems des NAD(P)⁺-Cofaktors, umfassend Methylacetoacetat und NAD(P)⁺-abhängige Alkoholdehydrogenase (ADH).

2. Verfahren nach Anspruch 1, wobei die NAD(P)⁺-abhängige HSDH eine Enzymaktivität im Bereich von 0,25 bis 12 U aufweist.

3. Verfahren nach Anspruch 1, wobei die Verbindungen der Formeln (I), (II) oder (III), deren Salze oder Derivate eine Konzentration im Bereich von 0,5 % bis 4 % (Gewicht/Volumen) aufweisen.

4. Verfahren nach Anspruch 1, wobei die Verbindungen der Formeln (I), (II) oder (III), deren Salze oder Derivate eine Konzentration im Bereich von 12,5 mM bis 100 mM aufweisen.

5. Verfahren nach einem der vorstehenden Ansprüche, wobei der NAD(P)⁺-Cofaktor in katalytischen Mengen in einer Konzentration im Bereich von 0,2 mM bis 0,8 mM verwendet wird.

6. Verfahren nach einem der vorstehenden Ansprüche, wobei das Methylacetoacetat-Co-Substrat in einer Menge verwendet wird, die gleich 2 Äquivalenten bezogen auf die jeweiligen Verbindungen (I), (II) und (III) ist.

7. Verfahren nach einem der vorstehenden Ansprüche, wobei das Methylacetoacetat-Co-Substrat in Mengen im Bereich von 0,3 % bis 2,4 % (Volumen/Volumen) verwendet wird.

8. Verfahren nach einem der vorstehenden Ansprüche, wobei die Oxidation quantitativ, d.h. höher oder gleich 99,5 % ist.

9. Verfahren nach einem der vorstehenden Ansprüche, durchgeführt bei Raumtemperatur, d.h. bei ca. 20 °C.

10. Verfahren nach einem der vorstehenden Ansprüche, durchgeführt bei einem pH-Wert von 8.

11. Verfahren nach Anspruch 10, ferner umfassend ein Puffersystem, vorzugsweise bestehend aus einem Kaliumphosphatpuffer.

12. Verfahren nach Anspruch 1, **dadurch gekennzeichnet, dass** es in einem wässrig/organischen Zweiphasensystem durchgeführt wird, wobei das organische Lösungsmittel vorzugsweise Isopropylacetat ist.

13. Verfahren nach den Ansprüchen 11 und 12, wobei das Puffersystem ein Kaliumphosphatpuffer und das organische Lösungsmittel Isopropylacetat ist.

14. Verfahren nach Anspruch 1, wobei das Salz der Cholsäure oder der Chenodesoxycholsäure Natriumsalz ist.

15. Verfahren zur Herstellung von Ursodesoxycholsäure (IV), **dadurch gekennzeichnet, dass** es ein Verfahren zur Synthese von 3α,12α-Dihydroxy-7-Keto-5β-Cholansäure (IV) nach einem beliebigen der Ansprüche 1 bis 14 umfasst.

16. Verfahren zur Herstellung von Ursodesoxycholsäure (IV), **dadurch gekennzeichnet, dass** es ein Verfahren zur Synthese von 3α,7α-Dihydroxy-12-Keto-5β-Cholansäure (VI) nach einem der Ansprüche 1 bis 14 umfasst.

17. Verfahren zur Herstellung von Ursodesoxycholsäure (IV), **dadurch gekennzeichnet, dass** es ein Verfahren zur Synthese von 3α-Hydroxy-7,12-Keto-5β-Cholansäure (VII) nach einem der Ansprüche 1 bis 14 umfasst.

18. Verfahren zur Herstellung von Ursodesoxycholsäure (IV), **dadurch gekennzeichnet, dass** es ein Verfahren zur Synthese von 7-Keto-Lithocholsäure (IX) nach einem der Ansprüche 1 bis 14 umfasst.

19. Verfahren zur Herstellung von Ursodesoxycholsäure (IV), **dadurch gekennzeichnet, dass** es ein Verfahren zur Synthese von 3α,12α-Dihydroxy-7-Keto-5β-Cholansäuremethylester (VIII) nach einem der Ansprüche 1 bis 13 umfasst.

## Revendications

1. Procédé d'oxydation enzymatique régiosélective au niveau de positions 7 et/ou 12 de composés ayant la formule : 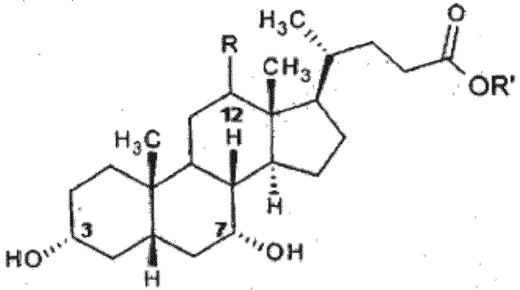
(I) Acide cholique R = OH ; R' = H
(II) Ester méthylique d'acide cholique R = OH ; R' = CH3
(III) Acide chénodésoxycholique R = H ; R' = H
ou des sels ou dérivés de ceux-ci, comprenant la réaction desdits composés avec de l'hydroxystéroïde déshydrogénase (HSDH) dépendant de NAD(P)*, en présence d'un système de régénération du cofacteur NAD(P)* comprenant de l'acétoacétate de méthyle et de l'alcool déshydrogénase (ADH) dépendant de NAD(P)*.

2. Procédé selon la revendication 1, dans lequel le HSDH dépendant de NAD(P)* * a une activité enzymatique dans la plage de 0,25 à 12 U.

3. Procédé selon la revendication 1, dans lequel les composés ayant la formule (I), (II) ou (III), des sels ou dérivés de ceux-ci ont une concentration dans la plage de 0,5% à 4% (poids/volume).

4. Procédé selon la revendication 1, dans lequel les composés ayant la formule (I), (II) ou (III), des sels ou dérivés de ceux-ci ont une concentration dans la plage de 12,5 mM à 100 mM.

5. Procédé selon l'une quelconque des revendications précédentes, dans lequel le cofacteur NAD(P)* est utilisé en quantités catalytiques, à une concentration dans la plage de 0,2 mM à 0,8 mM.

6. Procédé selon l'une quelconque des revendications précédentes, dans lequel l'acétoacétate de méthyle de co-substrat est utilisé dans une quantité égale à 2 équivalents rapportés à chacun des composés (I), (II) et (III).

7. Procédé selon l'une quelconque des revendications précédentes, dans lequel l'acétoacétate de méthyle de co-substrat est utilisé dans des quantités dans la plage de 0,3% à 2,4% (volume/volume).

8. Procédé selon l'une quelconque des revendications précédentes, dans lequel l'oxydation est quantitative, c'est-à-dire supérieure ou égale à 99,5%.

9. Procédé selon l'une quelconque des revendications précédentes, exécuté à température ambiante, c'est-à-dire à environ 20°C.

10. Procédé selon l'une quelconque des revendications précédentes, exécuté à un pH égal à 8.

11. Procédé selon la revendication 10, comprenant en outre un système tampon, comprenant de préférence un tampon de phosphate de potassium.

12. Procédé selon la revendication 1, **caractérisé en ce qu'**il est exécuté dans un système diphasique aqueux/organique, dans lequel le solvant organique est de préférence de l'acétate d'i-propyle.

13. Procédé selon les revendications 11 et 12, dans lequel le système tampon est un tampon de phosphate de potassium et le solvant organique est de l'acétate d'i-propyle.

14. Procédé selon la revendication 1, dans lequel le sel d'acide cholique ou d'acide chénodéoxycholique est du sel de sodium.

15. Procédé de préparation d'acide ursodéoxycholique (IV), **caractérisé en ce qu'**il comprend un procédé pour la synthèse d'acide 3α,12α-dihydroxy-7-céto-5β-cholanoïque (IV) selon l'une quelconque des revendications 1 à 14.

16. Procédé de préparation d'acide ursodéoxycholique (IV), **caractérisé en ce qu'**il comprend un procédé pour la synthèse d'acide 3α,7α-dihydroxy-12-céto-5β-cholanoïque (VI) selon l'une quelconque des revendications 1 à 14.

17. Procédé de préparation d'acide ursodéoxycholique (IV), **caractérisé en ce qu'**il comprend un procédé pour la synthèse d'acide 3α-hydroxy-7,12-céto-5β-cholanoïque (VII) selon l'une quelconque des revendications 1 à 14.

18. Procédé de préparation d'acide ursodéoxycholique (IV), **caractérisé en ce qu'**il comprend un procédé pour la synthèse d'acide 7-céto-lithocholique (IX) selon l'une quelconque des revendications 1 à 14.

19. Procédé de préparation d'acide ursodéoxycholique (IV), **caractérisé en ce qu'**il comprend un procédé pour la synthèse d'ester méthylique d'acide 3α,12α-dihydroxy-7-céto-5β-cholanoïque (VIII) selon l'une quelconque des revendications 1 à 13.
